# EUROPEAN PATENT SPECIFICATION

(11) **EP 4 559 502 B1**
(45) Date of publication and mention of the grant of the patent: **18.11.2026**
(21) Application number: 23842439.4
(22) Date of filing: 21.07.2023
(51) Int. Cl.: A61M 16/08, A61M 16/00

(54) **VENTILATION ASSEMBLY AND VENTILATION DEVICE**
LÜFTUNGSANORDNUNG UND LÜFTUNGSVORRICHTUNG
ENSEMBLE DE VENTILATION ET DISPOSITIF DE VENTILATION

(30) Priority: 21.07.2022 CN 202210874262
(43) Date of publication of application: 28.05.2025
(73) Proprietor: BMC Medical Co., Ltd., Beijing 100089 (CN)
(72) Inventor: JING, Bowei, Beijing 100089 (CN); ZHUANG, Zhi, Beijing 100089 (CN); GUO, Xiaoshuai, Beijing 100089 (CN)
(74) Representative: Grünecker Patent- und Rechtsanwälte PartG mbB
(86) International application number: PCT/CN2023/108701
(87) International publication number: WO 2024/017377

(56) References cited:
- EP-B1- 1 773 434
- WO-A1-2017/006189
- WO-A1-2022/141536
- WO-A1-2022/141536
- WO-A1-2022/142881
- WO-A1-2022/142881
- CN-A- 110 711 300
- CN-A- 110 711 300
- CN-A- 112 587 775
- CN-A- 115 252 986
- CN-U- 206 652 062
- CN-U- 214 807 562
- CN-U- 214 807 562
- CN-U- 219 290 361
- KR-A- 20140 017 924
- KR-A- 20140 017 924
- US-A1- 2004 035 422
- US-A1- 2005 112 013
- US-A1- 2005 166 921
- US-B2- 11 141 552

## Description

### Technical field

The embodiments of the present disclosure relate to the technical field of ventilation device, in particular to a ventilation assembly and ventilation device.

### Technical background

A ventilation device helps a patient breathe by providing positive pressure gas to open the patient's trachea. In order to make the air enter the patient's lungs when inhaling, and lung gas can be discharged smoothly when exhaling, it is necessary to adjust output pressure and flow rate of ventilation device in real time according to the patient's breathing phase.

In the prior art, the ventilation device comprises a fan, an air discharging cavity connected with the air discharging port of the fan, and an ventilation tube connected to the air discharging cavity, and the output pressure and flow rate are adjusted by adjusting rotating speed of the fan.

US 2004/0035422 A1 a pressure support system with enhanced noise reduction comprising a pressure generator, specifically a blower assembly having an inlet and an outlet, a first conduit that connects a gas source, such as ambient atmosphere, to the inlet of the pressure generator; a second conduit coupled to the outlet of the pressure generator to deliver the flow of gas to a patient; and a valve assembly, wherein the valve assembly communicates gas from the second conduit to the first conduit.

CN 214807562 U discloses a ventilation treatment device with a system for self-disinfection, comprising a host, a power unit, a heating unit, and an external disinfection pipeline located outside the host housing, wherein the two ends of the disinfection pipeline are detachably connected to the host inlet and the host outlet, respectively.

CN 112587775 A discloses a ventilation treatment device with a disinfection function, comprising a host for generating treatment gas, which has a host air inlet and a host air outlet, and a disinfection device for generating high-temperature disinfection gas, wherein the disinfection device is connected to the host's air inlet or air outlet to introduce the hot gas into the host's air path for disinfection.

EP 1 773 434 B1 discloses a method and apparatus for reducing noise in a Roots-type blower, particularly the noise generated by flow back, comprising a housing defining a rotor chamber with an inlet and an outlet, two inter-meshing rotors that move gas in gas transport chambers from the inlet to the outlet, and at least one outlet gas flow channel formed in the inner surface of the housing, wherein the outlet gas flow channel extends from the outlet back into the rotor chamber.

However, in a process of adjustment, the gas in the air discharging cavity is prone to turbulence, which leads to noise in the air discharging cavity.

### Summary of the invention

The present disclosure provides a ventilation assembly and ventilation device, which are used to solve a problem that the gas in a air discharging cavity is prone to generate turbulence during a adjustment of a fan, resulting in noise in the air discharging cavity.

On the one hand, the present disclosure provides a ventilation assembly, which comprises an air blowing apparatus for sucking gas from an air suction port and discharging same from an air discharging port; a housing internally provided with: an air feeding cavity in communication with the air suction port of the air blowing apparatus and provided with an air inlet in communication with the outside; an air discharging cavity in communication with the air discharging port of the air blowing apparatus and used for being connected to a ventilation tube worn by a patient; and a connecting cavity that allows the air feeding cavity to be in communication with the air discharging cavity, so that part of gas in the air discharging cavity flows back to the air suction port of the air blowing apparatus by means of the connecting cavity and the air feeding cavity;
wherein the connecting cavity comprises a plurality of connecting subcavities, the connecting subcavities are arranged in parallel along a backflow direction of gas.

Optionally, when the connecting cavity comprises a plurality of connecting subcavities, at least two of the connecting subcavities are connected in parallel and at least two of the connecting subcavities are connected in series.

Optionally, a partition plate is arranged between the connecting cavity and the air discharging cavity, and a partition plate is arranged between the connecting cavity and the air feeding cavity, and the partition plate is provided with a vent hole.

Optionally, a diversion hole is arranged between two adjacent connecting subcavities, and the gas in the air discharging cavity enters into the air feeding cavity through the vent hole and the diversion hole.

Optionally, the housing further comprises an installation cavity, and the air blowing apparatus is installed in the installation cavity, and the installation cavity and the air feeding cavity are separated by a first partition plate, and a damping element of the air blowing apparatus passes through the first partition plate and is connected to a side wall of the air feeding cavity.

Optionally, the air discharging cavity is provided with an air outlet connected with the ventilation tube, and the air discharging port and the air outlet are staggered.

On the other hand, the present disclosure provides a ventilation device, which comprises a ventilation assembly.

In the ventilation assembly and ventilation device provided by the present disclosure, an air feeding cavity, an air discharging cavity and a connecting cavity are arranged in a housing, an air suction port of an air blowing apparatus is connected with the air feeding cavity, an air discharging port of the air blowing apparatus is connected with the air discharging cavity, and the connecting cavity is connected with the air feeding cavity and the air discharging cavity. After the connecting cavity connects with the air feeding cavity and the air discharging cavity, because a pressure of the air discharging cavity is greater than that of the air feeding cavity, part of gas in the air discharging cavity can enter the air feeding cavity through the connecting cavity, and then be sucked into the air blowing apparatus through the air suction port. That is to say, a gas path of pressure relief is formed from the air discharging cavity to the air discharging port of the air blowing apparatus. When the gas in the air discharging cavity has pressure fluctuation or turbulence, an influence of the pressure fluctuation or turbulence can be alleviated through the gas path of pressure relief, thus reducing noise.

The above description is only an overview of the technical scheme of the present disclosure, which can be implemented according to the contents of the specification in order to clearly understand the technical means of the present disclosure, and in order to make the above and other purposes, features and advantages of the present disclosure more obvious and understandable, the following is specific embodiments of the present disclosure.

### Brief description of drawings

In order to explain the embodiments of the present disclosure or the technical scheme in the prior art more clearly, the appended drawings needed to be used in the description of the embodiments or the prior art will be briefly introduced below. Apparently, the appended drawings in the following description are some embodiments of the present disclosure, and other drawings can be obtained according to these appended drawings without expenditure of creative labor for persons skilled in the field.
FIG. 1 is a first sectional view of a ventilation assembly provided by an embodiment of the present disclosure;
FIG. 2 is a second sectional view of a ventilation assembly provided by an embodiment of the present disclosure;
FIG. 3 is a sectional view of an air feeding cavity and a connecting cavity in a ventilation assembly provided in tips of the present disclosure;
FIG. 4 is an enlarged view of a part at I in FIG. 3.

### Reference numeral:

100- air blowing apparatus;
110- air discharging port;
120- air suction port;
130- damping element;
200- housing;
210- air discharging cavity;
211- air outlet;
220- connecting cavity;
221- first connecting subcavity;
222- second connecting subcavity;
223- diversion hole;
230- air feeding cavity;
231- vent hole.

### Detailed Description

In the following, the technical scheme in the embodiments of the present disclosure will be clearly and completely described with reference to the appended drawings in the embodiments of the present disclosure. Apparently, the described embodiments are a part of the embodiments of the present disclosure, but not the whole embodiments. Based on the embodiments in the present disclosure, all other embodiments obtained by persons skilled in this field without expenditure of creative labor belong to the protection scope of the present disclosure.

A ventilation device refers to a device that can provide positive pressure gas to open the patient's trachea, thus helping the patient to breathe. The ventilation device can be a ventilation treatment device, such as a ventilator. The ventilation device usually includes a ventilation assembly, a ventilation tube and a control apparatus.

One end of the ventilation tube is worn on the patient to communicate with an airway of the patient, and the other end is connected to the ventilation assembly. Wherein, the ventilation tube can be worn on the patient's face through a breathing mask or inserted into the patient's respiratory tract, and the present disclosure does not limit a wearing mode of the ventilation tube.

The ventilation assembly can suck in external air and discharge the external air into the ventilation tube.

The control apparatus is connected with the ventilation assembly, and when in use, the control apparatus controls the ventilation assembly to suck in external air, and discharge the external air into the patient's trachea through the ventilation tube at a certain output pressure and a certain output flow rate, so as to open the patient's trachea and help the patient breathe.

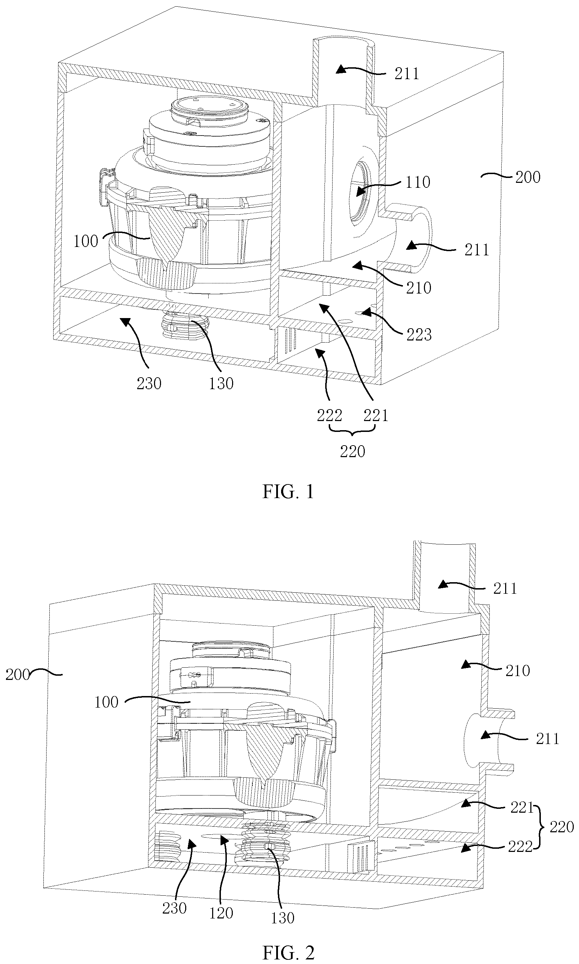

As shown in FIG. 1 and FIG. 2, the ventilation assembly includes a housing 200 and an air blowing apparatus 100.

The air blowing apparatus 100 is used to suck gas from the suction port 120 and discharge it from the air discharging port 110. The air blowing apparatus 100 can be connected with a control apparatus (not shown in the figure) to adjust the output pressure and output flow rate under control of the control apparatus.

Optionally, the blowing apparatus 100 includes a fan, which is connected with the control apparatus, and the output pressure and output flow rate of the blowing apparatus 100 are adjusted by adjusting rotating speed of the fan.

Inside the housing 200, there provided with an air feeding cavity 230, an air discharging cavity 210 and a connecting cavity 220. The air suction port 120 of the air blowing apparatus 100 connects with the air feeding cavity 230, and the air discharging port 110 of the air blowing apparatus 100 connects with the air discharging cavity 210. The air blowing apparatus 100 can suck gas from the air feeding cavity 230 and discharge the gas into the air discharging cavity 210.

The air feeding cavity 230 may be provided with an air inlet in communication with the outside, and the air discharging cavity 210 may be provided with an air outlet 211, which is connected with the ventilation tube, so that the air blowing apparatus 100 can suck in external gas (such as, air) entering the air feeding cavity 230 from the air inlet and discharge the external gas into the ventilation tube through the air outlet 211.

The air discharging port 110 and the air outlet 211 are arranged in a staggered manner, so that the gas entering the air discharging cavity 210 from the air discharging port 110 can be prevented from directly entering the ventilation tube from the air outlet 211, so that the air discharging cavity 210 can maintain a certain pressure.

When the ventilation assembly works, the air blowing apparatus 100 sucks gas from the air feeding cavity 230 and discharges the gas into the air discharging cavity 210, so the air discharging cavity 210 is a high-pressure cavity and the air feeding cavity 230 is a low-pressure cavity. In actual use, on the one hand, when flow rate and pressure of the gas discharged from the air discharging port 110 of the air blowing apparatus 100 fluctuate, it is easy to cause the housing 200 to vibrate, thus generating noise. On the other hand, an internal structure of the air discharging cavity 210 is likely to cause the gas discharged by the air blowing apparatus 100 to generate turbulence in the air discharging cavity 210, and the turbulence causes the housing 200 to vibrate, thus generating noise. That is to say, during use, noise is easily generated in the air discharging cavity 210.

The connecting cavity 220 connects with the air feeding cavity 230 and the air discharging cavity 210, so that part of the gas in the air discharging cavity 210 flows back to the air suction port 120 of the air blowing apparatus 100 through the connecting cavity 220 and the air feeding cavity 230. After the connecting cavity 220 connects with the air feeding cavity 230 and the air discharging cavity 210, because a pressure of the air discharging cavity 210 is greater than that of the air feeding cavity 230, part of the gas in the air discharging cavity 210 can enter the air feeding cavity 230 through the connecting cavity 220, and then be sucked into the air blowing apparatus 100 through the suction port 120. That is to say, a gas path of pressure relief is formed from the air discharging cavity 210 to the air suction port 120 of the air blowing apparatus 100. When pressure fluctuation or turbulence occurs in the gas in the air discharging cavity 210, the influence caused by the pressure fluctuation or turbulence can be alleviated by the gas path of pressure relief, thereby reducing noise.

Optionally, the connecting cavity 220 includes one or more connecting subcavities. When the connecting cavity 220 includes a connecting subcavity, the connecting subcavity connects with the air discharging cavity 210 and the air feeding cavity 230. When a plurality of connecting subcavities are provided, a structure of each connecting subcavity can be designed according to working conditions, and an arrangement mode of the plurality of connecting subcavities can also be designed according to the working conditions, so that a structure of the connecting cavity 220 is more flexible and can easily meet the actual use needs.

When a plurality of connecting subcavities are provided, a number of the connecting subcavities can be two, three, four, etc., which is not limited in the present disclosure.

A plurality of connecting subcavities are arranged in parallel along a backflow direction of the gas. After the plurality of connecting subcavities are arranged in parallel, it is equivalent that the gas path of pressure relief includes a plurality of gas subpaths of pressure relief arranged in parallel, and backflow gas of each gas subpath of pressure relief is adjusted by arranging the structure of each gas subpath of pressure relief, so that total backflow gas is more controllable.

Illustratively, the connecting cavity 220 includes two connecting subcavities, each of which connects with the air discharging cavity 210 and each of which connects with the air feeding cavity 230.

The two connecting subcavities can be connected or not connected. Not in accordance with the present claims, the plurality of connecting subcavities are arranged in series along the backflow direction of the gas. After the plurality of connecting subcavities are connected in series, pressure of the gas path of pressure relief between the air discharging cavity 210 and the air feeding cavity 230 can be gradually reduced, so that a pressure relief air flow is more stable.

Illustratively, along the gas backflow direction, the connecting cavity 220 includes a first connecting subcavity 221 and a second connecting subcavity 222, and a partition plate is arranged between the first connecting subcavity 221 and the second connecting subcavity 222, and a diversion hole 223 is arranged on the partition plate, and a shape of the diversion hole 223 can be grid-shaped, circular, triangular, polygonal and the like.

Optionally, at least two of the plurality of connecting subcavities are connected in parallel and at least two of the plurality of connecting subcavities are connected in series.

Illustratively, a number of the connecting subcavities is three, wherein two connecting subcavities are connected in parallel and then connected in series with another connecting subcavity. Certainly, the number of connecting subcavities can also be four, wherein two connecting subcavities are connected in parallel, and the remaining two connecting subcavities are connected in series, and the connecting subcavities connected in parallel are connected in series with the connecting subcavities connected in series.

Optionally, a partition plate is provided between the connecting cavity 220 and the air discharging cavity 210, and the partition plate is provided with a vent hole 231.

Optionally, a partition plate is provided between the connecting cavity 220 and the air feeding cavity 230, and the partition plate is provided with a vent hole 231.

By arranging the partition plate between two adjacent cavities and arranging the vent hole 231 on the partition plate, the pressures of the air discharging cavity 210, the connecting cavity 220 and the air feeding cavity 230 are gradually reduced, and the shape and position of the vent hole 231 can be arranged to make the backflow gas satisfy use conditions.

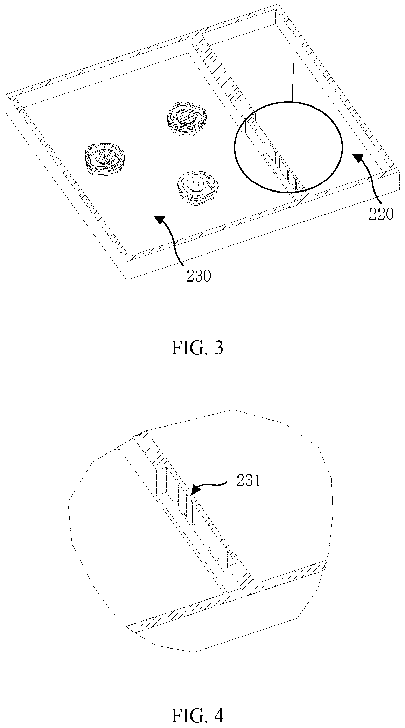

Illustratively, as shown in FIG. 3 and FIG. 4, the vent hole 231 is grid-shape. Certainly, the vent hole 231 may be circular, triangular, polygonal, etc.

When the housing 200 is provided with both the vent hole 231 and the diversion hole 223, the gas in the air discharging cavity 210 can enter the air feeding cavity 230 through the vent hole and the diversion hole 223.

Optionally, the housing 200 further includes an installation cavity in which the air blowing apparatus 100 is installed. After the air blowing apparatus 100 is installed in the installation cavity, the installation cavity can at least partially shield the noise generated by the air blowing apparatus 100.

Optionally, the installation cavity and the air feeding cavity 230 are separated by a first partition, and a support of the air blowing apparatus 100 passes through the first partition and is connected to a side wall of the air feeding cavity 230.

The first partition is used to separate the installation cavity from the air feeding cavity 230, so strength and rigidity of the first partition plate are weak. As an outermost part of the housing 200, the side wall of the air feeding cavity 230 is used not only to enclose the air feeding cavity 230, but also as a skeleton of the housing 200, so its strength and rigidity are strong. Connecting the air blowing apparatus 100 to the side wall of the air feeding cavity 230 can make the air blowing apparatus 100 fixed more firmly and reduce vibration in an operation process of the air blowing apparatus 100.

Illustratively, the blowing apparatus 100 includes three supports, which are located at three vertices of a triangle. This makes fixing of the air blowing apparatus 100 more firm.

Alternatively, the support is connected to the side wall of the air feeding cavity 230 by a damping element 130. The damping element 130 can buffer the vibration generated during the operation of the air blowing apparatus 100, thereby reducing influence of the vibration on the housing 200.

The damping element 130 can be a rubber pad or other damping elements, as long as it can buffer the vibration of the air blowing apparatus 100.

Finally, it should be noted that in this disclosure, relational terms such as first and second are only used to distinguish one entity or operation from another entity or operation, and do not necessarily require or imply any such actual relationship or order between these entities or operations. Moreover, the terms "including", "containing" or any other variation thereof are intended to cover non-exclusive inclusion, so that a process, method, article or terminal device including a series of elements includes not only those elements, but also other elements not explicitly listed, or elements inherent to such process, method, article or terminal device. Without more restrictions, the element defined by the phrase "including one" does not exclude that there are other identical elements in the process, method, article or terminal device including the element.

## Claims

1. A ventilation assembly, comprising:
an air blowing apparatus (100) for sucking gas from an air suction port and discharging same from an air discharging port (110);
a housing (200) internally provided with:
an air feeding cavity (230) in communication with the air suction port (120) of the air blowing apparatus (100) and provided with an air inlet in communication with the outside;
an air discharging cavity (210) in communication with the air discharging port (110) of the air blowing apparatus (100) and used for being connected to a ventilation tube worn by a patient;
and a connecting cavity (220) that allows the air feeding cavity (230) to be in communication with the air discharging cavity (210), so that part of gas in the air discharging cavity (210) flows back to the air suction port (120) of the air blowing apparatus (100) by means of the connecting cavity (220) and the air feeding cavity (230);
wherein the connecting cavity (220) comprises a plurality of connecting subcavities,
**characterized in that**
the connecting subcavities are arranged in parallel along a backflow direction of gas.

2. The ventilation assembly according to claim 1, wherein when the connecting cavity comprises a plurality of connecting subcavities, at least two of the connecting subcavities are connected in parallel and at least two of the connecting subcavities are connected in series.

3. The ventilation assembly according to claim 1, wherein a partition plate is arranged between the connecting cavity (220) and the air discharging cavity (210), and a partition plate is arranged between the connecting cavity (220) and the air feeding cavity (230), and the partition plate is provided with a vent hole (231).

4. The ventilation assembly according to claim 3, wherein a diversion hole (223) is arranged between two adjacent connecting subcavities (221, 222), and the gas in the air discharging cavity (210) enters into the air feeding cavity (230) through the vent hole (231) and the diversion hole (223).

5. The ventilation assembly according to claim 1, wherein the housing (200) further comprises an installation cavity, and the air blowing apparatus (100) is installed in the installation cavity, and the installation cavity and the air feeding cavity (230) are separated by a first partition plate, and a damping element (130) of the air blowing apparatus (100) passes through the first partition plate and is connected to a side wall of the air feeding cavity (230).

6. The ventilation assembly according to claim 1, wherein the air discharging cavity (210) is provided with an air outlet (211) connected with the ventilation tube, and the air discharging port (110) and the air outlet (211) are arranged in a staggered manner.

7. A ventilation device comprising a ventilation assembly according to any one of claims 1 to 6.

## Patentansprüche

1. Lüftungsanordnung, umfassend:
eine Gebläsevorrichtung (100) zum Ansaugen von Gas aus einer Luftansaugöffnung und zum Ausblasen desselben aus einer Luftausblasöffnung (110);
ein Gehäuse (200), das im Inneren Folgendes aufweist:
eine Luftzufuhrkammer (230), die mit der Luftansaugöffnung (120) der Gebläsevorrichtung (100) in Strömungsverbindung steht und mit einem Lufteinlass versehen ist, der mit der Außenumgebung in Strömungsverbindung steht;
eine Luftabgabekammer (210), die mit der Luftausblasöffnung (110) der Gebläsevorrichtung (100) in Strömungsverbindung steht und dazu vorgesehen ist, mit einem von einem Patienten getragenen Beatmungsschlauch verbunden zu werden;
und eine Verbindungskammer (220), die eine Strömungsverbindung zwischen der Luftzufuhrkammer (230) und der Luftabgabekammer (210) ermöglicht, sodass ein Teil des Gases in der Luftabgabekammer (210) mittels der Verbindungskammer (220) und der Luftzufuhrkammer (230) zu der Luftansaugöffnung (120) der Gebläsevorrichtung (100) zurückströmt;
wobei die Verbindungskammer (220) eine Vielzahl von Verbindungsteilkammern umfasst,
**dadurch gekennzeichnet, dass**
die Verbindungsteilkammern entlang einer Rückströmrichtung des Gases parallel angeordnet sind.

2. Lüftungsanordnung nach Anspruch 1, wobei dann, wenn die Verbindungskammer eine Vielzahl von Verbindungsteilkammern umfasst, wenigstens zwei der Verbindungsteilkammern parallel verbunden sind und wenigstens zwei der Verbindungsteilkammern in Reihe verbunden sind.

3. Lüftungsanordnung nach Anspruch 1, wobei eine Trennplatte zwischen der Verbindungskammer (220) und der Luftabgabekammer (210) angeordnet ist und eine Trennplatte zwischen der Verbindungskammer (220) und der Luftzufuhrkammer (230) angeordnet ist und die Trennplatte mit einer Überströmöffnung (231) versehen ist.

4. Lüftungsanordnung nach Anspruch 3, wobei eine Umlenköffnung (223) zwischen zwei benachbarten Verbindungsteilkammern (221, 222) angeordnet ist und das Gas in der Luftabgabekammer (210) durch die Überströmöffnung (231) und die Umlenköffnung (223) in die Luftzufuhrkammer (230) eintritt.

5. Lüftungsanordnung nach Anspruch 1, wobei das Gehäuse (200) ferner eine Einbaukammer umfasst und die Gebläsevorrichtung (100) in der Einbaukammer eingebaut ist und die Einbaukammer und die Luftzufuhrkammer (230) durch eine erste Trennplatte getrennt sind und ein Dämpfungselement (130) der Gebläsevorrichtung (100) durch die erste Trennplatte hindurchtritt und mit einer Seitenwand der Luftzufuhrkammer (230) verbunden ist.

6. Lüftungsanordnung nach Anspruch 1, wobei die Luftabgabekammer (210) mit einem Luftauslass (211) versehen ist, der mit dem Beatmungsschlauch verbunden ist, und die Luftausblasöffnung (110) und der Luftauslass (211) versetzt angeordnet sind.

7. Lüftungsvorrichtung, umfassend eine Lüftungsanordnung nach einem der Ansprüche 1 bis 6.

## Revendications

1. Ensemble de ventilation comprenant :
un appareil de soufflage d'air (100) destiné à aspirer du gaz depuis un orifice d'aspiration d'air et à le refouler par un orifice de refoulement d'air (110) ;
un boîtier (200) comprenant intérieurement :
une cavité d'alimentation en air (230) en communication fluidique avec l'orifice d'aspiration d'air (120) de l'appareil de soufflage d'air (100) et pourvue d'une entrée d'air en communication fluidique avec l'extérieur ;
une cavité de refoulement d'air (210) en communication fluidique avec l'orifice de refoulement d'air (110) de l'appareil de soufflage d'air (100) et destinée à être reliée à un tuyau de ventilation porté par un patient ;
et une cavité de liaison (220) permettant une communication fluidique entre la cavité d'alimentation en air (230) et la cavité de refoulement d'air (210), de sorte qu'une partie du gaz contenu dans la cavité de refoulement d'air (210) reflue vers l'orifice d'aspiration d'air (120) de l'appareil de soufflage d'air (100) par l'intermédiaire de la cavité de liaison (220) et de la cavité d'alimentation en air (230) ;
dans lequel la cavité de liaison (220) comprend une pluralité de sous-cavités de liaison,
**caractérisé en ce que**
les sous-cavités de liaison sont disposées en parallèle suivant une direction de retour du gaz.

2. Ensemble de ventilation selon la revendication 1, dans lequel, lorsque la cavité de liaison comprend une pluralité de sous-cavités de liaison, au moins deux des sous-cavités de liaison sont reliées en parallèle et au moins deux des sous-cavités de liaison sont reliées en série.

3. Ensemble de ventilation selon la revendication 1, dans lequel une plaque de séparation est disposée entre la cavité de liaison (220) et la cavité de refoulement d'air (210), et une plaque de séparation est disposée entre la cavité de liaison (220) et la cavité d'alimentation en air (230), et la plaque de séparation est pourvue d'un orifice de passage du gaz (231).

4. Ensemble de ventilation selon la revendication 3, dans lequel un orifice de déviation (223) est disposé entre deux sous-cavités de liaison adjacentes (221, 222), et le gaz contenu dans la cavité de refoulement d'air (210) pénètre dans la cavité d'alimentation en air (230) par l'orifice de passage du gaz (231) et l'orifice de déviation (223).

5. Ensemble de ventilation selon la revendication 1, dans lequel le boîtier (200) comprend en outre une cavité d'installation, et l'appareil de soufflage d'air (100) est installé dans la cavité d'installation, et la cavité d'installation et la cavité d'alimentation en air (230) sont séparées par une première plaque de séparation, et un élément d'amortissement (130) de l'appareil de soufflage d'air (100) traverse la première plaque de séparation et est relié à une paroi latérale de la cavité d'alimentation en air (230).

6. Ensemble de ventilation selon la revendication 1, dans lequel la cavité de refoulement d'air (210) est pourvue d'une sortie d'air (211) reliée au tuyau de ventilation, et l'orifice de refoulement d'air (110) et la sortie d'air (211) sont disposés de manière décalée.

7. Dispositif de ventilation comprenant un ensemble de ventilation selon l'une quelconque des revendications 1 à 6.
